# DEMANDE DE BREVET EUROPEEN

(11) **EP 3 842 517 A1**
(43) Date de publication de la demande: **30.06.2021**
(21) Numéro de dépôt: 20216603.9
(22) Date de dépôt: 22.12.2020
(51) Int. Cl.: C12N 1/20, A23C 9/12, A23C 19/032

(54) **LEVAINS BIOPROTECTEURS OBTENUS À PARTIR DE LAITS NATIFS : UTILISATION DE CES LEVAINS POUR PRÉPARER DES LEVAINS TERROIRS ET POUR PURIFIER LE LAIT CRU**

(30) Priorité: 23.12.2019 FR 1915420
(71) Demandeur: Flores de Terroirs, 17340 Chatelaillon Plage (FR)
(72) Inventeur: DUMARCHE, Claude, Louis, 17340 CHATELAILLON (FR); TAMBADOU, Fatoumata, 17000 LA ROCHELLE (FR)
(74) Mandataire: Lavoix

(57) **Abrégé**

La présente invention concerne un levain bioprotecteur comprenant plus de 99,00% de bactéries de l'espèce *Lactococcus lactis* en abondance relative, et obtenu par une méthode de préparation comprenant une étape de fermentation spontanée de lait natif de mammifère à une température comprise entre 20°C et 30°C, de préférence à 25°C, jusqu'à un pH d'arrêt de la fermentation compris entre 4,8 et 5,8, de préférence de 5,0.

## Description

La présente invention concerne la préparation de levains bioprotecteurs et l'utilisation de ceux-ci.

Le fromage et les produits laitiers sont produits et consommés depuis des millénaires pour leurs qualités gustatives et nutritionnelles. La production d'un fromage débute par la collecte et la préparation du lait qui reste un produit naturel fragile. Après la traite, le lait est stocké à des températures comprises entre 4 et 12°C (étape appelée report) pour limiter l'évolution d'éventuelles microflores pathogènes. En fonction de la durée du report, cette réfrigération modifie les proportions des différents groupes microbiens du lait. Elle sélectionne par exemple les bactéries à Gram négatif psychrotolérantes qui, par protéolyse et lipolyse, modifieront aussi les caractéristiques biochimiques des laits (Fricker et al. (2011) Int. J. Food Microb. 145 :S24-S30). Un report de lait à 12°C, malgré son effet amplificateur, devrait permettre en revanche de mieux respecter les équilibres initiaux des laits mais sans pour autant s'affranchir des pathogènes (Montel et al. (2012) Ecosystèmes des laits et des fromages au lait cru - enjeux pour leur maîtrise. Renc. Rech. Ruminants, 2012, 19). En absence de traitement du lait cru, sa qualité microbiologique a une importance cruciale pour la sécurité alimentaire et la qualité organoleptique des produits fabriqués, en particulier pour les AOP (Appellation d'Origine Protégée) majoritairement fabriqués à partir du lait cru.

Aujourd'hui, quatre genres bactériens pathogènes sont responsables de la majorité des cas de toxi-infection alimentaire dans les produits laitiers et fromagers : *Listeria monocytogenes, Salmonella* spp., *Staphylococcus aureus* producteurs d'entérotoxines, et les *Escherichia coli* producteurs de Shiga-Toxines (STEC) (Arques et al. (2015) BioMed Res. Int. 2015). Le lait cru constitue un milieu de culture potentiel pour la croissance de ces bactéries. Parmi ces pathogènes, seuls *L. monocytogenes, S. aureus* et *Salmonella* spp. sont soumis à des normes règlementaires européennes dans ces produits (Règlement CE 1441/2007). Malgré l'absence de critères microbiologiques applicables pour les STEC, l'obligation de garantir la sécurité des denrées alimentaires inscrite dans le « paquet hygiène » incite les industriels à rechercher ce pathogène pour garantir son absence.

Entre 2017 et 2019, 42 fromages fabriqués en France ont fait l'objet d'un rappel et d'une notification auprès du RASFF (« *Rapid Alert System for Food and Feed* ») pour des niveaux élevés de contaminations en *L. monocytogenes.*

De 2017 à 2019, 13 lots de fromages au lait cru et au lait pasteurisé fabriqués en France ont été rappelés avec une notification auprès du RASFF pour présence de *Salmonella* dans 25 g.

Entre 2017 et 2019, 33 lots de fromages fabriqués en France ont été retirés du marché après détection d'*E. coli* et d'*E*. *coli* STEC*.*

De même, plusieurs microorganismes sont impliqués dans l'altération du lait et des fromages. Ce sont, pour la plupart, des bactéries à Gram négatif psychrotrophes, des levures, des moisissures, des *lactobacilles* hétéro-fermentaires et des bactéries sporulés (Boor et al. (2017) J. Dairy Sci. 100 :9933-9951). Les bactéries psychrotrophes représentent une cause majeure d'altération des produits laitiers car elles font parties de la flore naturelle du lait et de son environnement. Les genres majoritaires rencontrés sont *Pseudomonas* (70% des psychrotrophes du lait), *Bacillus,* les coliformes et les entérobactéries.

Différents conservateurs chimiques de qualité alimentaire tels que le sorbate de potassium, le benzoate de potassium, la natamycine, les acides lactique et propioniques, sont utilisés pour éviter et prévenir la croissance indésirable de levures, moisissures et bactéries, et augmenter ainsi la durée de vie des produits laitiers. Même si l'utilisation de ces conservateurs est réglementée par l'Union Européenne, il existe actuellement une forte demande des consommateurs, soutenue par les autorités publiques, pour des produits naturels sans conservateurs chimiques et moins transformés.

La biopréservation ou bioprotection consiste à inoculer dans un produit des microorganismes sélectionnés et/ou leurs métabolites, de manière à inhiber la flore indésirable qui pourrait s'y trouver, et ainsi allonger la durée de vie et la sécurité des aliments crus. En diminuant la perte économique due à la contamination de produits alimentaires, la bioprotection pourrait remplacer l'utilisation de conservateurs chimiques et les traitements thermiques. Elle permettrait une conservation naturelle des aliments en préservant leurs propriétés organoleptiques et nutritionnelles, souvent perdues sous l'effet des agents chimiques ou de la chaleur.

Les bactéries lactiques (LAB) présentent un potentiel de développement important dans les procédés de bioprotection car elles bénéficient du statut GRAS (« *Generally Recognized as Safe* ») aux Etats-Unis et pour plusieurs d'entre elles, du statut QPS (« *Qualified Presumption of Safety* ») en Europe. Plusieurs études ont montré leur potentiel inhibiteur sur des flores pathogènes ou d'altération dans de nombreux produits alimentaires (saumon, fromages, charcuterie, produits végétaux, bière...). Le pouvoir antimicrobien des bactéries lactiques est attribué à divers facteurs comme la compétition nutritionnelle et la production d'un ensemble de métabolites possédant des propriétés antimicrobiennes. Il s'agit principalement de la production d'acides faibles (acide lactique, acide acétique), de peroxyde d'hydrogène, de dioxyde de carbone, de diacétyle (le 2,3-butanedione), de reutérine ou de bactériocines.

La demande internationale WO2013/153074 décrit une composition antifongique comprenant une combinaison de *Lactobacillus rhamnosus* et de *Lactobacillus paracasei* pour augmenter la bioprotection contre les levures.

La demande internationale WO2012/136830 décrit également une composition antifongique comprenant au moins un *Lactobacillus rhamnosus* et au moins un *Lactobacillus paracasei.*

Une culture commerciale FreshQ® constituée de *Lactobacillus rhamnosus* et de *Lactobacillus paracaseiis,* ayant une activité antagoniste contre les levures et moisissures des produits laitiers fermentés est disponible chez Chr. Hansen, Danemark. Parmi les autres solutions bioprotectrices antifongiques sur le marché des produits laitiers, on peut également citer Lyofast LPRA de SACCO et Aroma-Prox®RP80 de BIOPROX. Ces deux produits contiennent une combinaison de *Lactobacillus plantarum* et *Lactobacillus rhamnosus.*

Le brevet français FR2933272 décrit pour sa part un procédé de préparation de levain à partir de lait cru (procédé Bionatif®). Cette solution ne prend toutefois en charge que les laits crus contaminés en flores d'altération. Elle ne permet pas de prendre l'ensemble des laits crus contaminés à la fois en flores pathogènes et flores d'altération. De plus, il s'affranchit d'une partie de la diversité microbienne du fait de trop nombreux repiquages successifs et de l'apport de compléments nutritionnels autres que le lait natif.

Ainsi, les cultures protectrices sur le marché sont constituées de souches domestiques sélectionnées et cultivées sur milieux gélosés, ou elles présentent des propriétés antagonistes ciblant au maximum 2 microorganismes (bactéries, levures ou moisissures). Pourtant plusieurs pathogènes et flores d'altération peuvent être présent en même temps dans le lait cru. Par conséquent, il existe un réel besoin industriel de développer des cultures bioprotectrices possédant une activité antibactérienne à la fois contre les flores pathogènes et les flores d'altération afin d'apporter une sécurité sanitaire au lait cru et de limiter l'utilisation des conservateurs chimiques.

A la connaissance des inventeurs, il n'existe pas à ce jour de méthode permettant d'éliminer à la fois les flores pathogènes et les flores d'hygiène du lait cru. Le seul moyen de limiter la contamination du lait est d'utiliser et de respecter les Bonnes Pratiques d'Hygiène à la traite et de respecter les températures pour le stockage et le transport du lait. Même en appliquant des mesures d'hygiène rigoureuses lors la production de lait et tout au long de la chaîne alimentaire, les microorganismes indésirables continuent leur cycle cellulaire car le lait est un milieu favorable au développement de ces germes. De plus, le développement de ces microorganismes indésirables impacte également la qualité microbiologique des laits crus en terme de germes totaux, ce qui amène à qualifier les laits crus de laits paucimicrobiens. Or, les flores microbiennes du lait cru sont les agents majeurs de l'élaboration des produits laitiers fermentés. Elles interviennent au niveau technologique, au niveau sensoriel et au niveau hygiénique. Ainsi, la disparition de l'effet de barrière exercée par la flore technologique favorise l'implantation des flores pathogènes.

Le défi pour les filières au lait cru est donc de préserver la diversité microbienne ayant un intérêt technologique tout en éliminant les microorganismes indésirables.

La présente invention répond à ce besoin.

La présente invention résulte de la découverte inattendue par les inventeurs qu'il était possible de produire, par fermentation spontanée à partir de laits natifs de mammifère, des levains ayant des activités bioprotectrices à l'encontre à la fois de flores d'altération et de flores pathogènes du lait.

La présente invention concerne ainsi un levain bioprotecteur comprenant plus de 99,00% de bactéries de l'espèce *Lactococcus lactis* en abondance relative, et obtenu par une méthode de préparation comprenant une étape de fermentation spontanée de lait natif de mammifère à une température comprise entre 20°C et 30°C, de préférence à 25°C, jusqu'à un pH d'arrêt de la fermentation compris entre 4,8 et 5,8, de préférence de 5,0.

La présente invention a également pour objet une méthode de préparation d'un levain à partir de lait natif de mammifère, ladite méthode comprenant :
a) une étape de fermentation spontanée de lait natif de mammifère, à une température comprise entre 20°C et 30°C, de préférence à 25°C, jusqu'à un pH d'arrêt de la fermentation à 6,
b) une étape d'ajout du levain bioprotecteur selon l'invention dans le lait fermenté obtenu à la fin de l'étape a), et
c) une étape de fermentation spontanée du lait fermenté obtenu à la fin de l'étape b), à une température comprise entre 20°C et 30°C, de préférence à 25°C, jusqu'à un pH d'arrêt de la fermentation compris entre 4,8 et 5,8, de préférence de 5,0.

Un autre objet de la présente invention concerne une méthode de réduction ou d'élimination de la flore pathogène et de la flore d'altération d'un lait cru de mammifère, ladite méthode comprenant le mélange dudit lait cru avec le levain bioprotecteur selon l'invention.

La présente invention concerne également l'utilisation du levain bioprotecteur selon l'invention dans la fabrication d'un fromage, d'un beurre, d'un lait fermenté ou d'une crème fermentée.

La présente invention a également pour objet une méthode de préparation d'une morge comprenant le mélange de flores d'affinage avec le levain bioprotecteur selon l'invention.

Un autre objet de l'invention concerne le levain bioprotecteur selon l'invention pour son utilisation comme probiotique.

### Description détaillée de l'invention

### Définitions

Par « levain », on entend ici une culture de micro-organismes issue du lait natif destinée à être introduite dans des produits à fermenter en vue de maîtriser la fermentation.

Par « lait cru de mammifère », on entend ici le lait produit par la sécrétion de la glande mammaire d'animaux d'élevage et non chauffé à plus de 40 ºC, ni soumis à un traitement d'effet équivalent.

Par « lait natif de mammifère », on entend ici le lait ayant été prélevé à la ferme tel qu'il est sorti de la mamelle juste après la traite et n'ayant subi aucun traitement physique ou thermique, ni report au froid, autre que la congélation des échantillons entre -20 et - 45°C.

Le lait peut provenir de n'importe quel type de mammifère, tel qu'une vache, une brebis, une chèvre, une bufflonne, une chamelle, une jument ou une biche. Le lait produit par des mammifères sains est souvent considéré comme étant pauvre en microorganismes en sortie de mamelle, la composition de son microbiote est, par conséquent, directement liée à l'environnement de la ferme (peau des trayons, équipements de production, alimentation...).

Le terme « ufc » signifie « unité formant colonie » et est l'unité de mesure généralement reconnue par l'homme du métier pour quantifier les bactéries capables de former une colonie.

Par « fermentation spontanée », on entend ici la fermentation du lait natif de mammifère amorcée par les microorganismes présents naturellement dans le lait natif. La fermentation spontanée est ainsi typiquement amorcée par les bactéries lactiques naturellement présentes dans le lait natif.

Dans le contexte de l'invention, le terme « bactéries lactiques » (LAB) désigne des bacilles ou des coques à Gram positif, immobiles, non sporulés, micro-aérophiles ou anaérobies, catalase négatif capables de produire de l'acide lactique suite à la fermentation des sucres. Les bactéries lactiques peuvent être ajoutées dans le lait sous forme de ferments ou être naturellement présentes. Sur le plan technologique, les principaux genres utilisés sont *Lactococcus* spp, *Streptococcus* spp., *Lactobacillus* spp., *Leuconostoc* spp., *Pediococcus* spp., *Brevibacterium* spp. et *Enterococcus* spp.

Par « flore technologique », on entend ici l'ensemble de la flore du lait natif participant à la maturation, à l'acidification, à la coagulation du lait cru et au développement de la flore de surface. La flore technologique comprenant par exemple les lactocoques, notamment *Lactococcus lactis, Lactococcus diacetylactis,* les lactobacilles et les bactéries du genre *Leuconostoc.*

Par « flore indésirable », on entend ici l'ensemble de la flore du lait contribuant à l'altération soit des propriétés organoleptiques (flore d'altération), soit de la qualité sanitaire (flore pathogène) du produit laitier final.

La flore d'altération comprend notamment les bactéries du genre *Pseudomonas* et les bactéries coliformes.

Par « bactérie conforme », on entend ici des bactéries Gram négatives fermentant le lactose avec production de gaz à 35-37°C en 48h, non-sporulantes, donnant une réponse négative au test à l'oxydase, aérobies ou anaérobies facultatives.

La flore pathogène comprend en particulier les bactéries *Listeria spp,* plus particulièrement *Listeria monocytogenes, Salmonella* spp., *Staphylococcus aureus* producteurs d'entérotoxines, et les *Escherichia coli* producteurs de Shiga-toxines (STEC), en particulier de sérotype 0157 :H7.

### Levain bioprotecteur

Par « bioprotection » ou « bioprotecteur », on entend ici la capacité à réduire ou éliminer, partiellement ou totalement, une flore indésirable présente dans un produit donné. Un levain bioprotecteur selon l'invention peut ainsi par exemple réduire la croissance, le nombre ou la concentration, de flores indésirables présentes dans une matrice par rapport à la croissance, le nombre ou la concentration de ces flores indésirables dans une matrice ne contenant pas ou n'ayant pas été mis en contact avec ce levain bioprotecteur.

Dans un mode de réalisation particulier, le levain bioprotecteur selon l'invention a une activité bioprotectrice à l'encontre de la flore pathogène et de la flore d'altération telles que définies dans la section « *Définitions* » ci-dessus.

Dans un mode de réalisation particulier, le levain bioprotecteur selon l'invention a une activité bioprotectrice à l'encontre de la flore pathogène, en particulier à l'encontre de *Listeria spp.,* (plus particulièrement *Listeria monocytogenes*) *Salmonella spp., Escherichia coli* STEC et *Staphylococcus aureus,* et de la flore d'altération, en particulier à l'encontre des coliformes, des laits crus et/ou natifs de mammifère.

Le levain bioprotecteur selon l'invention comprend plus de 99,00% de bactéries de l'espèce *Lactococcus lactis* en abondance relative.

Par « *Lactococcus lactis* », on entend une espèce de bactérie à Gram positif, non motile, non-sporulante, mesurant en moyenne de 0.5 à 1.5 µm.

Par « abondance relative », on entend ici la quantité relative au nombre d'individus d'une espèce donnée par unité de surface ou de volume par rapport au nombre total d'individus de toutes espèces confondues.

Dans le cadre de l'invention, l'abondance relative des espèces de bactéries présentes dans le levain bioprotecteur est de préférence déterminée par séquençage du gène codant pour la région V3-V4 de l'ARNr 16S après extraction de l'ADN présent dans le levain, et évaluation des abondances relatives des OTUs (Unité Opérationnelle Taxonomique) comme décrit dans Westcott et Schloss (2015) PeerJ 3 :e1487.

Dans un mode de réalisation particulier, le levain bioprotecteur selon l'invention comprend plus de 99,00% de bactéries de l'espèce *Lactococcus lactis* en abondance relative, de préférence plus de 99,20%, plus de 99,50%, plus de 99,90% ou 100% de bactéries de l'espèce *Lactococcus lactis* en abondance relative.

Dans un mode de réalisation particulier, le levain bioprotecteur selon l'invention comprend 99,24% de bactéries de l'espèce *Lactococcus lactis* en abondance relative.

Dans un autre mode de réalisation particulier, le levain bioprotecteur selon l'invention comprend 99,90% de bactéries de l'espèce *Lactococcus lactis* en abondance relative.

Dans un autre mode de réalisation particulier, le levain bioprotecteur selon l'invention comprend 100% de bactéries de l'espèce *Lactococcus lactis* en abondance relative.

Dans un mode de réalisation particulier, le levain bioprotecteur selon l'invention comprend plus de 99,00% de bactéries de l'espèce *Lactococcus lactis* en abondance relative, et éventuellement des bactéries du genre *Enterococcus,* des bactéries du genre *Hafnia* et/ou des bactéries du genre *Macrococcus.*

Par « bactérie du genre *Enterococcus* », on entend ici des coques à métabolisme aéro-anaérobie, dites cocci à Gram positif sans activité catalase, non mobiles, généralement sous forme de chainettes. Le genre *Enterococcus* inclut typiquement les espèces *Enterococcus faecalis, Enterococcus faecium* et *Enterococcus durans.* De préférence, les bactéries du genre *Enterococcus* présentes dans le levain bioprotecteur selon l'invention ne sont pas des bactéries pathogènes.

Par « bactérie du genre *Hafnia* », on entend ici des bactéries de la famille des entérobactéries incluant typiquement les espèces *Hafnia alvei* et *Hafnia paralvei.*

Par « bactérie du genre *Macrococcus* », on entend ici des cocci à Gram positif appartenant à la famille des Staphylococcaceae, non-motiles, non-sporulants, coagulase négatifs et catalase positifs. Le genre *Macrococcus* inclut typiquement les espèces *Macrococcus bovicus, Macrococcus brunensis, Macrococcus canis, Macrococcus carouselicus, Macrococcus caseolyticus, Macrococcus equipercicus, Macrococcus hajekii* et *Macrococcus lamae.*

Dans un mode de réalisation particulier, le levain bioprotecteur selon l'invention comprend plus de 99,00% de bactéries de l'espèce *Lactococcus lactis* en abondance relative, des bactéries du genre *Enterococcus* et des bactéries du genre *Hafnia.*

Dans un autre mode de réalisation particulier, le levain bioprotecteur selon l'invention comprend plus de 99,00% de bactéries de l'espèce *Lactococcus lactis* en abondance relative et des bactéries du genre *Macrococcus.*

Dans un mode de réalisation particulier, le levain bioprotecteur selon l'invention comprend :
(i) 99,24% de bactéries de l'espèce *Lactococcus lactis* en abondance relative, 0,69% de bactéries du genre *Enterococcus* en abondance relative et 0,06% de bactéries du genre *Hafnia* en abondance relative,
(ii) 100,00% de bactéries de l'espèce *Lactococcus lactis* en abondance relative,
(iii) 99,90% de bactéries de l'espèce *Lactococcus lactis* en abondance relative et 0,10% de bactéries du genre *Macrococcus* en abondance relative, ou
(iv) Un mélange de (i) et (ii), de (i) et (iii), de (ii) et (iii), ou de (i), (ii) et (iii).

De préférence, le levain bioprotecteur selon l'invention comprend entre 10⁸ ufc de bactéries /ml de levain et 10⁹ ufc de bactéries /ml de levain.

Du fait de sa méthode de préparation, le levain bioprotecteur selon l'invention est constitué uniquement de souches environnementales qui apportent la même sécurité alimentaire que les ferments composés de souches domestiques.

Par « souches environnementales », on entend ici la biodiversité dans des environnements naturels tels que les laits crus, les fourrages, les trayons ou le matériel de traite artisanale. Ces souches sont non cultivées sur milieux gélosés. Elles ont une grande diversité génétique et sont capables de se développer sur différents substrats sans entrer en compétition. Le travail collectif des souches environnementales aboutit à une multifonctionnalité associative qui serait responsable de la typicité, la richesse des profils aromatiques et l'intensité sensorielle des fromages fabriqués à partir de cette biodiversité.

A l'inverse, le terme « souches domestiques » désigne des bactéries isolées de ferments lactiques utilisés dans l'industrie laitière, cultivés sur milieux gélosés et ajoutés au lait dans le but de remplir différentes fonctions comme l'acidification maitrisée, le métabolisme du citrate et la protéolyse primaire. Ces souches présentent une faible diversité génétique, et dans un environnement technologique, on assiste à une compétition entre ces différentes souches aboutissant à une fonctionnalité dominante.

Le levain bioprotecteur selon l'invention est obtenu par une méthode de préparation comprenant une étape de fermentation spontanée de lait natif de mammifère à une température comprise entre 20°C et 30°C jusqu'à un pH d'arrêt de la fermentation compris entre 4,8 et 5,8.

La fermentation spontanée est mise en œuvre à une température comprise entre 20°C et 30°C, de préférence entre 21°C et 29°C, entre 22°C et 28°C, entre 23°C et 27°C, entre 24°C et 26°C, plus préférentiellement à 25°C. La mise en œuvre de la fermentation spontanée à cette température a l'avantage de favoriser au maximum la biodiversité des levains en permettant le développement de la flore technologique sans pour autant la sélectionner.

La fermentation spontanée est mise en œuvre jusqu'à un pH d'arrêt de la fermentation compris entre 4,8 et 5,8, de préférence entre 4,9 et 5,7, entre 4,9 et 5,6, entre 4,9 et 5,5, entre 4,9 et 5,4, entre 4,9 et 5,3, entre 4,9 et 5,2, entre 4,9 et 5,1, plus préférentiellement un pH de 5,0. L'arrêt de la fermentation spontanée à ce pH a l'avantage de favoriser le développement des bactéries homofermentaires, hétérofermentaires, et des bactéries lactiques qui métabolisent efficacement le citrate.

Dans un mode de réalisation particulier, la méthode de préparation du levain bioprotecteur selon l'invention inclut, après l'étape de fermentation spontanée, une étape de report en congélation du levain, typiquement afin de stabiliser la fermentation.

Par « congélation », on entend ici toute technique visant à faire passer un produit à l'état solide par des techniques de refroidissement forcé, en abaissant la température du produit et la maintenant en dessous de 0°C. De préférence, le levain bioprotecteur selon l'invention est congelé à une température de -85°C. De préférence, le levain bioprotecteur selon l'invention est maintenu à l'état congelé pendant 24h à 48h, typiquement pour ralentir l'activité métabolique des bactéries.

Dans un mode de réalisation particulier, la méthode de préparation du levain bioprotecteur selon l'invention inclut, après l'étape de fermentation spontanée, une étape de concentration dudit levain bioprotecteur.

Par « concentration », on entend ici toute technique permettant d'augmenter le rapport entre la quantité d'un microorganisme et le volume du milieu qu'elle occupe.

Dans un mode de réalisation préféré, ladite étape de concentration est mise en œuvre par repiquages répétés successivement.

Par « repiquage », on entend ici l'action de prélever une petite partie d'une culture de microorganismes (de préférence d'un levain bioprotecteur selon l'invention), puis de la transplanter sur un lait où elle continue à croître. De préférence, le repiquage est mis en œuvre en ensemençant 10 ml d'une culture de microorganismes obtenue après fermentation spontané dans 1L de lait de manière stérile. La culture est ensuite de préférence incubée à une température de 25°C dans un bio-fermenteur avec un suivi de la cinétique d'acidification en parallèle pour arrêter la culture au pH défini.

Dans un mode de réalisation préféré, les repiquages sont répétés successivement au maximum 3 fois. Ceci permet d'éviter au maximum la perte de biodiversité dans le levain bioprotecteur.

Typiquement, l'étape de concentration permet de passer d'un levain bioprotecteur comprenant 10⁵ à 10⁶ ufc de bactéries /ml à un levain bioprotecteur comprenant entre 10⁸ et 10⁹ ufc de bactéries /ml.

Dans un mode de réalisation préféré, la méthode de préparation inclut, après l'étape de fermentation, une étape de report en congélation telle que définie ci-dessus dudit levain bioprotecteur et/ou une étape de concentration telle que définie ci-dessus dudit levain bioprotecteur, de préférence par repiquages répétés successivement, en particulier au maximum 3 fois.

### Méthode de préparation de levain

La présente invention concerne également une méthode de préparation d'un levain tel que défini dans la section « *Définitions* » ci-dessus à partir de lait natif de mammifère tel que défini dans la section « *Définitions* » ci-dessus, ladite méthode comprenant :
a) une étape de fermentation spontanée, telle que définie dans la section *« Définitions* » ci-dessus, de lait natif de mammifère, à une température comprise entre 20°C et 30°C, jusqu'à un pH d'arrêt de la fermentation à 6,
b) une étape d'ajout du levain bioprotecteur tel que défini dans la section « *Levain bioprotecteur* » ci-dessus, dans le lait fermenté obtenu à la fin de l'étape a), et
c) une étape de fermentation spontanée, telle que définie à la section *« Définitions* » ci-dessus, du lait fermenté obtenu à la fin de l'étape b), à une température comprise entre 20°C et 30°C, de préférence à 25°C, jusqu'à un pH d'arrêt de la fermentation compris entre 4,8 et 5,8, de préférence de 5,0.

De préférence, le lait natif de mammifère fermenté à l'étape a) a une numération initiale de la Flore Mésophile Aérobie Revivifiable comprise entre 500 et 20000 ufc/ml.

Par « Flore Mésophile Aérobie Revivifiable » ou « FMAR », on entend ici les niveaux de microflores totales ou toutes les bactéries capables de former des colonies sur milieu gélosé à 30°C en aérobiose.

Si le lait natif de mammifère fermenté à l'étape a) est contaminé par une flore indésirable telle que définie à la section « *Définitions* » ci-dessus, cette contamination est de préférence comprise entre 0 et 20 ufc/ml pour les bactéries du genre *Listeria,* entre 0 et 20 ufc/ml pour les bactéries du genre *Salmonella*, entre 0 et 300 ufc/ml pour les bactéries de l'espèce *Escherichia coli,* entre 0 et 300 ufc/ml pour les bactéries de l'espèce *Staphylococcus aureus,* entre 0 et 100 ufc/ml pour les coliformes et/ou entre 0 et 50 ufc/ml pour les bactéries du genre *Pseudomonas.*

La fermentation spontanée est mise en œuvre à l'étape a) à une température comprise entre 20°C et 30°C, de préférence entre 21°C et 29°C, entre 22°C et 28°C, entre 23°C et 27°C, entre 24°C et 26°C, plus préférentiellement à 25°C. La mise en œuvre de la fermentation spontanée à cette température a l'avantage de favoriser au maximum la biodiversité du lait fermenté en permettant le développement de la flore technologique sans pour autant la sélectionner.

La fermentation spontanée est mise en œuvre à l'étape a) jusqu'à un pH d'arrêt de la fermentation égal à 6.

De préférence, à l'issue de l'étape a), le lait fermenté comprend de l'ordre de 10⁶ ufc/ml de population bactérienne.

A l'étape b), du levain bioprotecteur tel que défini dans la section « *Levain bioprotecteur »* ci-dessus, est ajouté au lait fermenté obtenu à l'étape a). Ledit levain bioprotecteur peut être ajouté par toute technique appropriée bien connue de l'homme du métier. Typiquement, le levain bioprotecteur est ajouté par ensemencement direct et stérile du lait fermenté. De préférence, en particulier lorsque le levain bioprotecteur comprend entre 10⁸ et 10⁹ ufc de bactéries/ml, ledit levain bioprotecteur est ajouté au lait fermenté à une concentration comprise entre 0,0001% et 0,0003% (v/v). De préférence, une quantité de levain bioprotecteur correspondant à 10⁵ à 10⁷ ufc de bactéries, en particulier entre 10⁶ et 3×10⁶ ufc, est ajoutée pour 1L de lait fermenté.

La fermentation spontanée est mise en œuvre à l'étape c) à une température comprise entre 20°C et 30°C, de préférence entre 21°C et 29°C, entre 22°C et 28°C, entre 23°C et 27°C, entre 24°C et 26°C, plus préférentiellement à 25°C. La mise en œuvre de la fermentation spontanée à cette température a l'avantage de favoriser au maximum la biodiversité du levain en permettant le développement de la flore technologique sans pour autant la sélectionner.

La fermentation spontanée est mise en œuvre à l'étape c) jusqu'à un pH d'arrêt de la fermentation compris entre 4,8 et 5,8, de préférence entre 4,9 et 5,7, entre 4,9 et 5,6, entre 4,9 et 5,5, entre 4,9 et 5,4, entre 4,9 et 5,3, entre 4,9 et 5,2, entre 4,9 et 5,1, plus préférentiellement un pH de 5,0.

De préférence, le levain obtenu à l'issue du procédé de préparation selon l'invention a une FMAR comprise entre 600×10⁶ et 10⁹ ufc/ml.

### Utilisations agroalimentaires

Le levain bioprotecteur selon l'invention permettant de purifier les laits crus de mammifère, il est particulièrement utile dans de nombreuses applications agroalimentaires utilisant ces laits crus et/ou impliquant une fermentation lactique.

La présente invention concerne ainsi également une méthode de réduction ou d'élimination de la flore pathogène et de la flore d'altération telles que définies dans la section « *Définitions* » ci-dessus d'un lait cru de mammifère tel que défini dans la section *« Définitions* » ci-dessus, ladite méthode comprenant le mélange dudit lait cru avec le levain bioprotecteur tel que défini dans la section « *Levain bioprotecteur* » ci-dessus.

De préférence, la contamination initiale du lait cru traité est comprise entre 0 et 20 ufc/ml pour les bactéries du genre *Listeria,* entre 0 et 20 ufc/ml pour les bactéries du genre *Salmonella,* entre 0 et 150 ufc/ml pour les bactéries de l'espèce *Escherichia coli,* entre 0 et 300 ufc/ml pour les bactéries de l'espèce *Staphylococcus aureus,* entre 0 et 150 ufc/ml pour les coliformes et/ou entre 0 et 50 ufc/ml pour les bactéries du genre *Pseudomonas.*

De préférence, la flore pathogène et la flore d'altération sont statistiquement significativement réduites. De préférence, la flore pathogène et la flore d'altération sont réduites jusqu'à atteindre un niveau strictement inférieur à 1 ufc/ml pour les bactéries du genre *Listeria* (autrement dit une élimination), un niveau strictement inférieur à 1 ufc/ml pour les bactéries du genre *Salmonella* (autrement dit une élimination), un niveau strictement inférieur à 10 ufc/ml pour les bactéries de l'espèce *Escherichia coli,* un niveau strictement inférieur à 30 ufc/ml pour les bactéries de l'espèce *Staphylococcus aureus,* un niveau strictement inférieur à 10 ufc/ml pour les coliformes et/ou un niveau strictement inférieur à 10 ufc/ml pour les bactéries du genre *Pseudomonas.* De manière particulièrement préférée, la flore pathogène et la flore d'altération sont totalement éliminées.

Le levain bioprotecteur selon l'invention peut être ajouté au lait cru à traiter par toute technique appropriée bien connue de l'homme du métier. Typiquement, le levain bioprotecteur est ajouté par ensemencement du lait cru. De préférence, en particulier lorsque le levain bioprotecteur comprend entre 10⁸ et 10⁹ ufc de bactéries /ml, ledit levain bioprotecteur est ajouté au lait cru à une concentration comprise entre 0,1% et 0,35% (v/v). De préférence, une quantité de levain bioprotecteur correspondant à 10⁵ à 10⁷ ufc de bactéries, en particulier entre 10⁶ et 3×10⁶ ufc, est ajoutée par ml de lait cru.

De préférence, le levain bioprotecteur est laissé en contact avec le lait cru pendant toute la durée de traitement ultérieur du lait cru, typiquement dans des procédés de fabrication de fromage, beurre, lait fermenté ou crème fermentée, typiquement à une température comprise entre 30°C et 38°C (par exemple pendant la maturation avant emprésurage) ou à une température de 4°C (par exemple pendant le stockage du lait cru à la ferme), ou à une température comprise entre 12°C et 14°C (par exemple en pré-maturation sur site industriel). Pour un stockage du lait cru à la ferme et pour une pré-maturation le levain bioprotecteur est de préférence ajouté respectivement à une quantité de 10³ et 10⁵ à 10⁶ ufc par ml de lait cru.

Le levain bioprotecteur selon l'invention peut également être utilisé dans la fabrication d'un fromage, d'un beurre, d'un lait fermenté ou d'une crème fermentée.

Par « fromage », on entend ici un aliment obtenu à partir de lait coagulé ou de produits laitiers, comme la crème, puis d'un égouttage suivi ou non de fermentation et éventuellement d'affinage. Les fromages incluent en particulier, les fromages à pâte pressée, tels que les fromages à pâte pressée non cuite, les fromages à pâte pressée demi-cuite et les fromages à pâte pressée cuite ; les fromages à pâte molle, tels que les fromages à pâte molle à croute fleurie, les fromages à pâte molle à croute lavée et les fromages à pâte molle à croute naturelle, les fromages à pâte fraiche tels que les fromages à pâte fraiche, les fromages à pâte filée et les fromages à pâte fondue ; et les fromages à pâte persillée. De préférence, le fromage est un fromage au lait cru.

Par « beurre », on entend ici un produit laitier extrait par barattage de la crème issue du lait.

Par « lait fermenté », on entend ici un produit laitier obtenu par fermentation du lait, dont les levains sont vivants, actifs et abondants (sauf si le produit a subi un traitement thermique après la fermentation). Les laits fermentés incluent typiquement les yaourts, la crème fraiche, le lait fermenté concentré, le stragisto, le labneh, l'ymer, l'ylette, le caillé, le lait ribot, le dahi, le kéfir, le gros lait, le lassi, le skyr.

Par « crème fermentée », on entend ici le produit laitier obtenu par la fermentation de la crème, de la crème reconstituée ou recombinée, par l'action de microorganismes appropriés, ce qui entraine la réduction du pH sans coagulation.

De préférence, le fromage, beurre, lait fermenté ou crème fermentée sont préparés à partir de lait cru, tel que défini dans la section « *Définitions* » ci-dessus, pasteurisé ou thermisé, et/ou ne subissent pas de traitement thermique à l'issue de leur fabrication.

Par « lait pasteurisé », on entend ici un lait traité par pasteurisation, un traitement consistant à chauffer le lait à une température comprise entre 62°C et 88°C durant une durée déterminée, avant un refroidissement brusque, de manière à éliminer un nombre important de microorganismes.

Par « lait thermisé », on entend ici un lait traité par chauffage pendant au moins 15 secondes à des températures comprises entre 57°C et 68°C, tel que le lait présente après ce traitement une réaction positive au test de la phosphatase alcaline.

Le levain bioprotecteur selon l'invention peut être utilisé à différentes étapes de la fabrication de ces produits. Il peut ainsi être utilisé lors des étapes de fermentation de ces produits mais aussi lors des étapes d'affinage ou des étapes de traitement de post-contaminations sur des laits traités thermiquement ou sur des laits microfiltrés.

Ainsi, un autre objet de la présente invention concerne une méthode de préparation d'une morge comprenant le mélange d'une flore d'affinage avec le levain bioprotecteur selon l'invention.

Par « morge », on entend ici la solution (à base de saumure), de frottage utilisée en cave au cours de l'affinage dans le but d'apporter une certaine quantité de sel, pour obtenir un taux de sel final sur le fromage affiné variable selon la fabrication, et ensemencer les fromages en blanc avec des flores d'affinage qui permettent d'obtenir une croûte caractéristique à chaque fromage.

Par « flore d'affinage », on entend ici les microorganismes utilisés dans la période de maturation pendant laquelle le fromage repose et reçoit des soins à la cave ou dans un hâloir. La flore d'affinage inclut typiquement des levures telles que *Candida utilis, Kluyveromyces lactis* et *Debaryomyces hansenii,* des champignons du genre *Geotrichum* tels que *Geotrichum candidum,* des champignons du genre *Penicillium* tels que *Penicillium candidum, Penicillium camemberti, Penicillium roqueforti, Penicillium nalgiovense* et *Cylindrocarpon heteronema,* des corynébactéries telles que les bactéries du genre *Brevibacterium* (par exemple *B. linens*), les bactéries du genre *Arthrobacter* (par exemple *A. globiformis*) et les bactéries du genre *Microbacterium.*

Le levain bioprotecteur selon l'invention peut être mélangé avec la morge par toute technique appropriée bien connue de l'homme du métier. Typiquement, le levain bioprotecteur est mélangé avec la morge par ensemencement. De préférence, en particulier lorsque le levain bioprotecteur comprend entre 10⁸ et 10⁹ ufc de bactéries/ml s, ledit levain bioprotecteur est ajouté à la morge à une concentration comprise entre 0,5% et 1% (v/v).

### Autres utilisations

Etant constitué de souches environnementales qui, de par leurs multifonctionalités, leurs développements associatifs et leurs capacités de lyse tardive devraient être plus stable dans le microbiote intestinal, le levain bioprotecteur selon l'invention devrait agir de manière efficace comme probiotique.

La présente invention concerne ainsi également le levain bioprotecteur tel que défini dans la section « *Levain bioprotecteur* » pour son utilisation comme probiotique.

Par « probiotique », on entend ici un microorganisme vivant, qui, quand il est ingéré en quantité suffisante, exerce des effets positifs sur la santé, au-delà des effets nutritionnels traditionnels.

De par ses activités bioprotectrices, le levain selon l'invention peut également être utilisé pour traiter des élevages afin de les démédicaliser et ainsi réduire l'usage de médicaments tout en prévenant et/ou traitant des infections dues à des bactéries, en particulier du genre *Salmonella, Campylobacter* ou *E. coli* STEC par exemple.

Dans le cadre de la présente demande, le terme « comprenant » doit être interprété comme couvrant toutes les caractéristiques spécifiquement mentionnées, ainsi qu'éventuellement certaines non-spécifiées additionnelles. Par ailleurs, l'utilisation du terme « comprenant » décrit également le mode de réalisation dans lequel aucune caractéristique autre que celles spécifiques mentionnées n'est présente (*i.e.* « consistant en »)_{.}

La présente invention sera illustrée plus en détail par les figures et exemples ci-dessous.

### Brève description des figures

La **Figure 1** présente l'effet inhibiteur de levains sur la croissance de plusieurs pathogènes alimentaires.
La **Figure 2** montre l'effet bioprotecteur des levains selon l'invention sur la croissance de *L. monocytogenes, S. enteritidis, S. aureus, E. coli, E. coli* O157 :H7 et de coliformes.
La **Figure 3** présente l'effet d'une combinaison de deux levains selon l'invention en co-culture : co-culture A ou co-culture B sur la croissance de *L. monocytogenes, S. enteritidis, E. coli* O157 :H7 et *S*. *aureus.*
La **Figure 4** montre des histogrammes représentant l'abondance relative des OTUs obtenues pour 3 levains bioprotecteurs selon l'invention.
La **Figure 5** illustre le dénombrement d'*Escherichia coli* (UFC/g) sans et avec la présence d'un ferment issu du procédé Bionatif® ou un ferment Bioprotecteur ajouté lors de la fabrication d'un fromage pâte molle lactique.
La **Figure 6** illustre le dénombrement des coliformes (UFC/g) sans et avec la présence d'un ferment issu du procédé Bionatif® ou un ferment Bioprotecteur ajouté lors de la fabrication d'un fromage pâte molle lactique.
La **Figure 7** illustre le dénombrement des *S. aureus* (UFC/g) sans et avec la présence d'un ferment issu du procédé Bionatif® ou un ferment Bioprotecteur ajouté lors de la fabrication d'un fromage pâte molle lactique.

### Exemples

### Exemple 1 : Fermentation stabilisante de laits natifs

Les inventeurs ont disposé d'une centaine de laits natifs (chèvres, vaches et brebis) prélevés dans les terroirs de plusieurs zones fromagères en France, en Europe, aux USA et au Japon.

Des prélèvements de ces laits ont été analysés pour dénombrer la Flore totale présente à 30°C, les flores d'altération (coliformes, *E. coli*) et les flores pathogènes (*Listeria* spp., *Salmonella* spp., et *S. aureus*)*.* Tous ces laits natifs avaient une contamination résiduelle variable et une Flore Mésophile Aérobie Revivifiable (FMAR) variable.

Seuls les laits crus sans *Listeria* spp. et sans *Salmonella* spp. ont été utilisés par la suite. Ce tri a été réalisé pour éviter les contaminations croisées de ces germes dans les autres laits. Ensuite les laits crus ont été soumis à une fermentation spontanée sous CINAC (Thermo Fisher Scientific, France) à une température d'acidification de 25°C et un pH d'arrêt de chaque fermentation de 5.

Après fermentation, les gels natifs (correspondant aux laits coagulés par les bactéries lactiques naturellement présentes dans les laits natifs, obtenus après fermentation spontanée) obtenus sont stabilisés par congélation à -45°C pendant 24 à 48h.

Après analyses microbiologiques, les inventeurs ont identifié une cinquantaine de gels natifs ne présentant pas de flores pathogènes ni de flores d'altération à la lecture.

La fermentation permet de multiplier la flore lactique et les autres flores naturellement présentes dans ces laits natifs sans sélection. Elle permet également de voir l'évolution de toutes les flores présentes au départ dans le lait.

Le stockage des laits natifs au froid a permis non seulement de stabiliser les gels mais également de ralentir l'activité métabolique des bactéries présentes dans les gels natifs.

La température de fermentation de 25°C a été choisie pour favoriser au maximum la biodiversité des gels. Cette température permet le développement de la flore technologique ainsi que des autres flores présentes dans le lait natif sans pour autant les sélectionner.

Le pH d'arrêt choisi permet quant à lui le développement des bactéries homofermentaires, hétéro-fermentaires et de celles qui métabolisent efficacement le citrate.

### Exemple 2 : Screening du potentiel inhibiteur des gels natifs

L'activité antagoniste d'un consortia ou d'une souche de bactéries lactiques envers une souche cible peut être mise en évidence en milieu liquide ou en milieu gélosé selon l'application envisagée. Pour une application de protection alimentaire ou de surface, le milieu gélosé sera privilégié (Ryan et al. (1996) Appliedand Environ. Microbiol. 62612-619).

Un criblage de l'activité antagoniste des 50 gels a été effectué vis-à-vis de plusieurs souches cibles de collections internationales (Collection de l'Institut Pasteur (CIP) ou American Type Culture Collection (ATTC)) telles que *Listeria innocua, Salmonella* spp, *Staphylococcus aureus, Staphylococcus epidermidis, Escherichia coli, E. coli* 0157 : H7, *Bacillus subtilis, Bacillus cereus, Enterococcus faecalis et Pseudomonas aeruginosa.*

Les souches cibles ont été cultivées en Bouillon cœur cervelle (BHI) ou Bouillon Tryptone Soja (TSB) une nuit à 30°C ou 37°C sous agitation constante à 160 rpm.

L'activité inhibitrice des gels natifs contre les souches cibles a été testée *in vitro* par la méthode de diffusion en milieu gélosé. Des dilutions appropriées de 1/10 à 1/100^{ème} de chaque culture « overnight » des souches cibles ont été ajoutées dans 20 ml de gélose BHI en surfusion mélangée manuellement. Une fois la gélose prise en masse, 20 µl de chaque gel natif ont été déposés sous forme d'une goutte à la surface de la gélose. Après que les gouttes ont séché, les géloses ont été incubées à 30°C ou 37°C pendant 24 h. En parallèle, des témoins ont été réalisés avec du lait UHT amené au même pH que les gels testés. Pour chaque gel natif testé, les tests ont été faits en triplicatas et l'activité antagoniste a été évaluée par la mesure du diamètre de la zone d'inhibition.

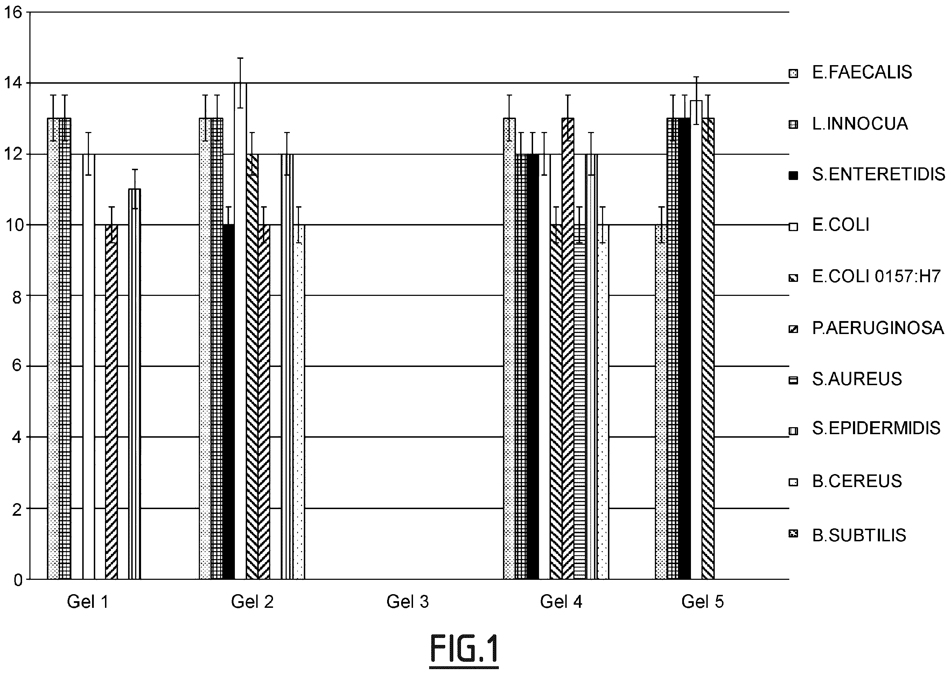

Les résultats présentés en **Figure 1** indiquent que certains gels natifs présentent une bonne capacité antagoniste de la croissance de plusieurs pathogènes dont *L. innocua, S. enteritidis, S. aureus, S. epidermidis, E. coli, E. coli* O157:H7, *B. subtilis, E. faecalis et P. aeruginosa.*

### Exemple 3 : Repiquage et concentration des gels natifs à activité bioprotectrice

Les 15 gels ayant les meilleures activités antagonistes envers les souches cibles ont été repiqués afin de les concentrer. En fonction des gels, plusieurs étapes de repiquages, mais pas plus de 3, ont été nécessaires.

Le fait de ne pas dépasser les 3 repiquages successifs permet d'éviter une perte de biodiversité. Ces repiquages ont pour but de vérifier que les quelques pathogènes et les coliformes dont *E. coli* contenus dans les laits natifs de départ ne se développent plus par réactivation.

Cette étape de concentration est également réalisée pour permettre l'utilisation du levain purifié en fabrication. Cette étape permet en effet de stabiliser le levain obtenu à une température de 25°C pour passer d'un gel natif comprenant 10⁵ à 10⁶ UFC/ml à un levain indéfini de fromagerie comprenant entre 10⁸ UFC/ml et 10⁹ UFC/ml.

L'analyse microbiologique des levains a montré un état sanitaire conforme aux législations internationales en vigueur.

### Exemple 4 : Criblage du potentiel bioprotecteur des levains natifs constitués d'un consortium complexe de souches environnementales

Des cultures de 100 ml de lait comprenant des levains bioprotecteurs selon l'invention et des souches cibles ont été incubées pendant une nuit à 28°C sans agitation.

L'effet inhibiteur potentiel de chaque levain a été testé sur plusieurs souches pathogènes et des souches d'altérations des produits laitiers. Le dénombrement de chaque souche cible avant et après une nuit d'incubation a été réalisé respectivement sur les milieux Rapid'L.mono (*L. monocytogenes*), VRBL (*S. enteritidis*), Baird-Parker (*S. aureus*) et gélose Coli ID (*E. coli* et *E. coli* O157:H7).

Dans cette étude, un ajout de 0,1 à 0,2% de levain dans 100 ml de lait inoculé soit avec 30 UFC/ml de *L. monocytogenes,* soit 30 UFC/ml de *S*. *enteritidis,* soit 400 UFC/ml de S. *aureus,* soit 300 UFC/ml de *E. coli,* soit 100 UFC/ml d'*E*. *coli* O157:H7 et 100 UFC/ml de coliformes a permis de réduire de façon significative et reproductible la croissance de ces souches cibles. Dans certains cas, les inventeurs ont observé une élimination complète et reproductible de l'ensemble des pathogènes testés après dénombrement sur milieux de cultures.

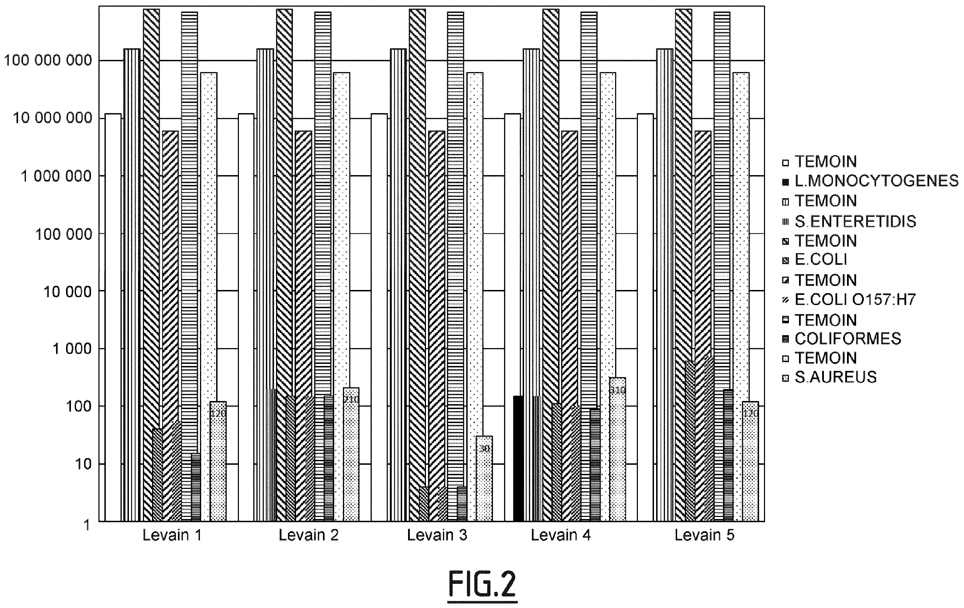

Les résultats présentés en **Figure 2** indiquent que toutes les souches cibles testées se développent très bien sur un milieu constitué de lait.

L'ajout de certains levains natifs pendant la fermentation du lait réduit fortement la croissance de bactéries pathogènes. Les levains issus de gels natifs présentent donc une bonne capacité antagoniste de la croissance des souches de *L. monocytogenes, S. enteritidis, S. aureus, E. coli, E. coli O157:H7 et de coliformes* (une réduction qui va jusqu'à - 8 log₁₀ de la population cible par comparaison au témoin sans levain).

Les levains 1 et 3 restent de bons candidats pour une activité de bioprotection, d'autant plus que ce sont des levains à large spectre d'activité antagoniste.

### Exemple 5 : Augmentation de l'effet bioprotecteur par une combinaison de coculture entre les levains

Après l'étape décrite dans l'Exemple 4, 5 levains ont été sélectionnés pour la suite de cette étude.

Les conditions expérimentales étaient identiques à celles décrites dans l'Exemple 4, à l'exception de ce qui suit : les levains bioprotecteurs ont d'abord été mis en co-culture entre eux à une température de 25°C et un pH d'arrêt de chaque fermentation de 5. Puis ces co-cultures ont été ajoutées à hauteur de 0,1% dans les laits inoculés avec les souches cibles sans agitation.

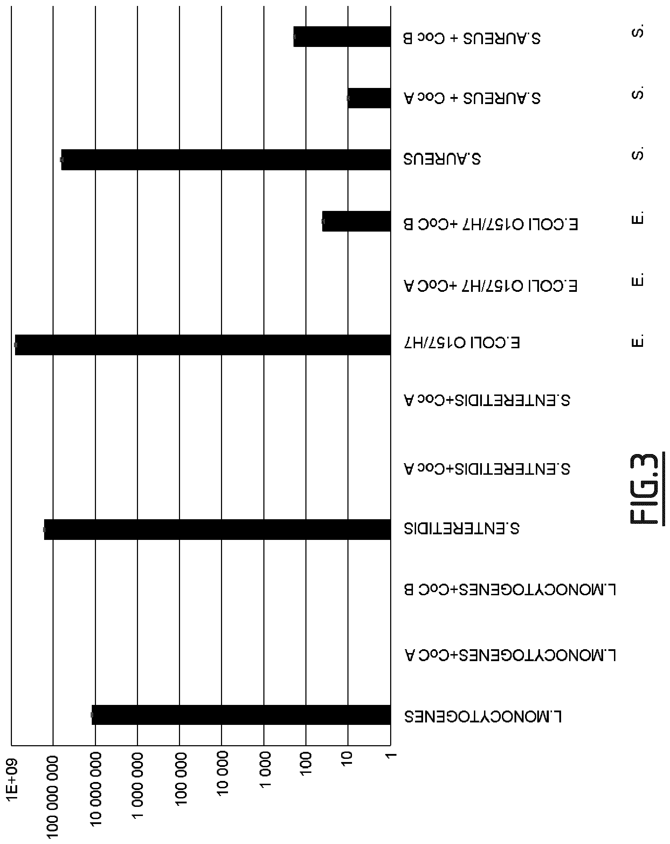

Les résultats sont présentés en **Figure 3****:**
- par comparaison au témoin sans co-culture, la coculture A permet d'une part d'éliminer la croissance de *L. monocytogenes, de S. enteritidis,* et *E. coli* O157:H7 et d'autre part de diminuer de 7 log₁₀ la croissance de *S*. *aureus* ;
- la coculture B permet d'éliminer la croissance de *L. monocytogenes et de S. enteritidis,* de diminuer de 7,5 log₁₀ la croissance d'*E*. *coli* O157:H7 et 6 log₁₀ la croissance de *S. aureus.*

L'assemblage de levains composés de souches environnementales permet donc de renforcer la bioprotection contre les pathogènes et permet également d'amplifier et d'élargir le spectre d'activité antagoniste envers les pathogènes. Cela démontre l'organisation collective, multifonctionnelle et intra-espèce des souches environnementales.

### Exemple 6 : Tests en laboratoire pilote pour optimiser l'apport à réaliser en fonction des process industriels.

L'avantage de l'utilisation des levains bioprotecteurs décrits ci-dessus est de pouvoir apporter dans les laits crus, les laits pasteurisés, les laits microfiltrés et thermisés mis en fabrication pour des technologies fromagères, beurrières et autres produits fermentés, une biodiversité environnementale appartenant déjà au lait cru sans perturber ni modifier le process industriel établi.

Les co-cultures utilisées dans l'exemple 4 ont été testées à différentes températures.

Les conditions expérimentales sont identiques à celles décrites dans l'exemple 4, les cocultures ont été ajoutées à raison de 0,1 à 0,3%. Ces levains bioprotecteurs sont efficaces à plusieurs températures notamment 4°C, 12°C, 20°C, 28°C et 35°C et ils peuvent donc être utilisés en maturation froide et en maturation chaude. L'efficacité accrue des levains bioprotecteurs à 4°C et à 12°C devrait permettre un début de purification des laits à la source, juste après récolte du lait cru. Les levains bioprotecteurs décrits ici peuvent ainsi être utilisés pour purifier les laits crus ou directement en technologie fromagère. Pour cela, le lait natif a de préférence une flore technologique comprise typiquement entre 500 et 20000 UFC/mL. Si le lait natif est contaminé par des flores indésirables, cette contamination est de préférence comprise entre 0 et 20 UFC/ml pour *Listeria,* 0 et 20 UFC/ml pour *Salmonella,* 0 et 300 UFC/ml pour *Escherichia coli,* 0 et 300 UFC/ml de S. *aureus,* 0 et 100 UFC/ml de coliformes, 0 et 50 UFC/ml de *Pseudomonas.*

Il est important de souligner que le procédé Bionatif® décrit dans le brevet français FR2933272 ne permet de traiter que les laits crus contaminés en flores d'altération dont les coliformes et *E. coli.* Les levains bioprotecteurs décrits ici permettent en revanche de traiter la totalité des laits natifs contaminés en *Listeria,* en *Salmonella,* en *Escherichia coli* dont les *Escherichia coli* producteurs de Shiga-Toxines, en *Staphylococcus aureus* et en *Pseudomonas* (ce qui donne accès à une plus grande biodiversité utile en technologie fermentaire).

Pour atteindre la meilleure efficacité, les levains bioprotecteurs décrits ici sont utilisés de préférence à des pourcentages minimaux compris entre 0,1 et 0,3%, ce qui correspond à une variation de 10⁶ à 3.10⁶ UFC de bactéries /ml.

### Exemple 7 : Etude de la diversité bactérienne présente dans 3 levains bioprotecteurs.

La diversité bactérienne de 3 levains bioprotecteurs utilisés dans les exemples ci-dessus a été étudiée par séquençage du gène codant pour la région V3-V4 de l'ARNr 16S. Après extraction de l'ADN, la technologie de séquençage utilisée est l'Illumina Miseq.

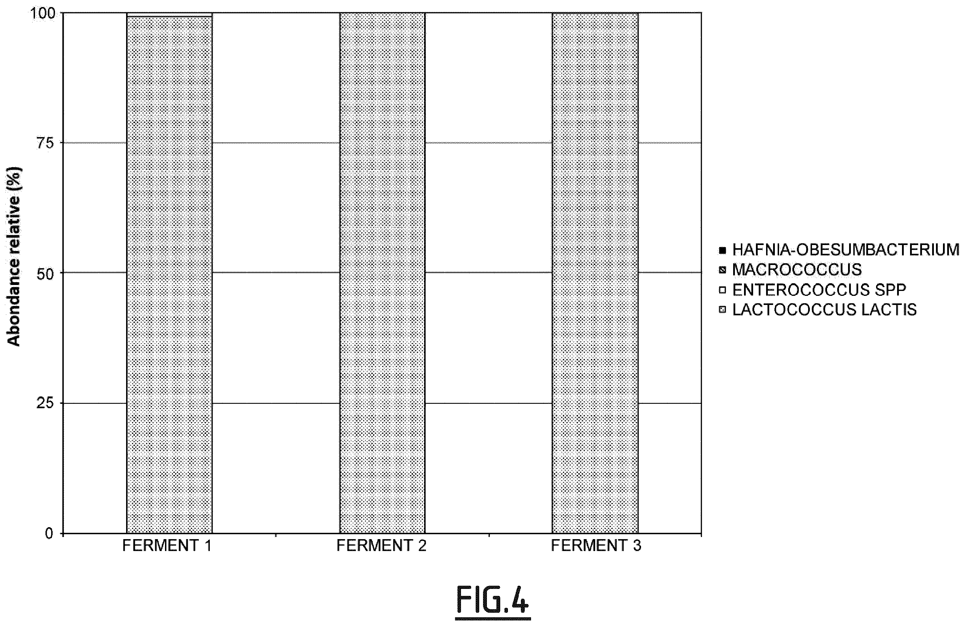

La **figure 4** représente l'abondance relative des OTUs (Unité Opérationnelle Taxonomique) obtenue suite au séquençage des 3 levains. Une OTU se définit comme un regroupement d'individus d'une même famille dont les séquences du gène codant pour l'ARNr 16S présentent plus de 97% d'homologie entre elle (Westcott & Schloss, (2015) PeerJ 3 :e1487).

Les 3 levains sont majoritairement composés de la famille des *Streptococaceae* et plus précisément de l'espèce *Lactococcus lactis.*

Le levain 1 est composé de l'espèce *Lactococcus lactis* (99,24% d'abondance relative), du genre *Enterococcus* spp (0,69% d'abondance relative) et du genre *Hafnia-Obesumbacterium* (0,06% d'abondance relative).

Le levain 2 est composé à 100% (abondance relative) de *Lactococcus lactis.*

Le levain 3 est composé de l'espèce *Lactococcus lactis* (99,9% d'abondance relative) et du genre *Macrococcus* (0,1% d'abondance relative).

Le levain 2 est le levain le plus bioprotecteur.

### Exemple 8 : Préparation d'un levain à partir d'un lait natif comprenant entre 500 et 20000 UFC/ml de Flore revivifiable.

Au vu des résultats significatifs obtenus sur l'activité inhibitrice, une autre application de ces levains bioprotecteurs a été envisagée par les inventeurs. Cette application consiste à utiliser les levains bioprotecteurs décrits ici pour préparer un levain terroir à partir de lait natif spécifique d'une zone géographique et purifiée par ces levains bioprotecteurs.

Le lait natif utilisé a été prélevé dans des exploitations laitières spécifiques d'une zone géographique et sélectionnées en fonction d'un Cahier des Charges spécifique. Ce Cahier des Charges permet de détecter les exploitations riches en flores endogènes situées dans un environnement, le mode d'élevage extensif, l'état sanitaire des troupeaux, l'alimentation et la richesse floristique de l'environnement étant pris en compte. Ces différents éléments permettent de sélectionner des laits natifs dont la composition microbienne reflète le terroir de la ferme ou de la zone d'exploitation. Au maximum 6 fermes ont été sélectionnées en fonction du nombre de producteurs. Après sélection, 2 litres de laits natifs ont été prélevés dans chaque ferme et conservés à -20°C jusqu'à l'étape de fermentation.

Une numération de la Flore Aérobie Mésophile Revivifiable, de la Flore technologique, de la flore d'altération et des flores pathogènes présentes a été effectuée pour avoir une indication prévisionnelle de l'aptitude technologique de ces laits natifs.

Le **tableau 1** représente les valeurs seuils de contamination résiduelle en UFC/ml de la numération des différentes flores des laits qui ont choisis pour la fermentation spontanée.

**Tableau 1**

| **Seuil de contamination en flores indésirables des laits natifs en UFC/ml** | |
|---|---|
| *Escherichia coli* | 0-300 |
| *Escherichia coli* STEC | 0-100 |
| *Coliformes* | 0-200 |
| Listeria spp | 0-20 |
| *Pseudomonas spp* | 0-50 |
| *Salmonella* spp | 0-20 |
| *Staphylococcus* a coagulasse positive | 0-300 |

Ces laits natifs ont de préférence une numération de la Flore Mésophile Aérobie Revivifiable (FMAR) de l'ordre de 500 à 20000 UFC/ml. Les laits sont ensuite acidifiés spontanément à 25°C sous agitation dans un fermenteur sans ajout intentionnel de ferments lactiques, d'antiseptiques ou d'inhibiteurs chimiques. Afin de purifier le plus efficacement possible un levain terroir, les inventeurs ont sélectionné tous les laits natifs s'acidifiant à 25°C à un pH supérieur ou égal à 5,50 après 16 à 30h d'acidification spontanée et un pH se stabilisant à environ 4,50 après 36 à 48h.

Les laits retenus après l'étape de fermentation spontanée ont été purifiés en utilisant le protocole suivant : 1 l de chaque lait natif a été mis en lactofermentation jusqu'à pH se stabilisant à 6 avec une population bactérienne de l'ordre de 10⁶ UFC/ml. A ce stade, 1 µL de levain bioprotecteur décrit ci-dessus a été ajouté dans le lait natif à pH 6. Ce mélange est laissé en lactofermentation naturelle à température de 25°C jusqu'à un arrêt à pH de stabilisation de 5.

La numération de la FMAR après cette étape indique une population de flore entre 600×10⁶ et 10⁹ UFC/ml selon les laits. Si les laits natifs de départ contenaient des flores pathogènes de type *Listeria, Salmonella* ou *E. coli* STEC, une numération systématique de ces pathogènes a été effectuée dans les laits après fermentation. La numération des flores d'altération a également été effectuée.

En absence de flores d'altération, une concentration de la FMAR a été effectuée en 3 cycles pour valider l'absence de flores d'altération résiduelles. En cas de détection de flores indésirables après cette étape de concentration, des fermentations stabilisantes à pH 5 ont été effectuées sans dépasser au maximum 3 fermentations pour préserver la biodiversité native des laits de départ. Un levain ainsi obtenu est constitué de souches environnementales reflétant la biodiversité microbienne d'un terroir avec une variabilité intraspécifique de *Lactococcus lactis.*

L'utilisation d'un tel levain en technologie laitière en particulier en technologie fromagère, beurrière et autres produits fermentés permet d'avoir une multifonctionnalité associative et stable des microorganismes dans l'écosystème de ces produits. Dans les produits, cette multifonctionnalité permet de mieux fixer la dynamique de l'eau de la phase colloïdale d'un lait puis du fromage en cours d'affinage. Cela apporte aux produits une typicité, une plus importante durée de vie du produit et une bonification de la texture.

### Exemple 9 : Exemple comparatif entre un ferment Bionatif® et un ferment Bioprotecteur sur une production de pâte molle lactique au lait cru.

Du lait cru frais de chèvre (traite du matin) a été acheté dans une ferme de la région puis transporté directement au laboratoire pour être utilisé le jour même. Des prélèvements ont été effectués pour mesurer le pH et dénombrer la Flore totale présente à 30°C, les flores d'altération (coliformes, *E. coli*) et les flores pathogènes (Listeria spp., Salmonella spp., *E*. *coli* STEC et *S*. *aureus*). Le lait cru est distribué dans des bouteilles stériles d'1L puis réchauffé à 22°C ± 1°C. Les ferments semi-direct (ferment Bionatif® et ferment Bioprotecteur) ont été ensemencés en double à 1% correspondant à une concentration de 5.10⁶ UFC. Le lait cru non ensemencé a été utilisé comme témoin positif. Les bouteilles ont été incubées pendant 5 heures dans un bain-marie à 22°C ± 1°C pour initier la maturation du lait cru jusqu'à un pH de 6,40 ± 0,05. Le lait est ensuite transféré dans des bols pour être emprésuré à hauteur de 50µL/L (présure 520 mg/L) pour un temps de coagulation de 45± 5 mn. Le caillé lactique obtenu a été moulé à la main au bout de 20 heures avec un égouttage progressif. Les fromages ont ensuite été conservés à 10°C ± 1°C (∼ 98% d'humidité relative) dans une cave à fromage pendant 30 jours pour imiter le vieillissement des fromages. Des échantillons ont été prélevés pour mesurer le pH et dénombrer la Flore totale, les coliformes, *E. coli* et *S*. *aureus* à l'ensemencement (T0h), avant emprésurage (T5h), au moulage (T20h), au démoulage (T48h) après 8 jours (T192h), 15 jours (T360h) et 30 jours (T720h) de vieillissement. Les résultats des dénombrements sont présentés dans les figures suivantes.

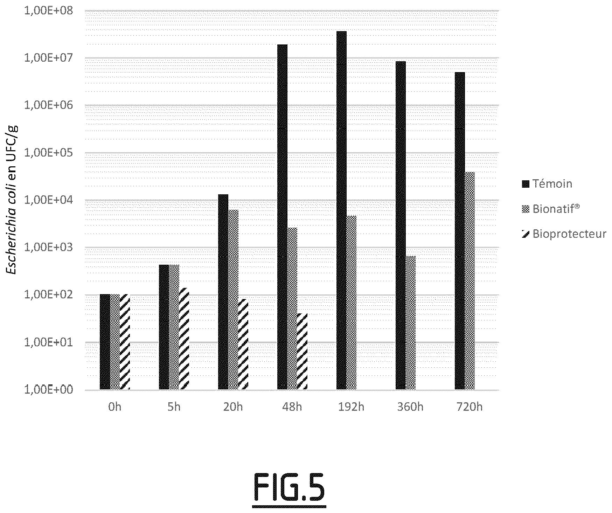

La **Figure 5** illustre le dénombrement d'*Escherichia coli* (UFC/g) sans et avec la présence d'un ferment issu du procédé Bionatif® ou un ferment Bioprotecteur ajouté lors de la fabrication d'un fromage pâte molle lactique.

Le lait cru utilisé contenait 102 UFC/mL d'*Escherichia coli* avant ensemencement des levains. Les *E. coli* se développent très bien dans les fromages aux laits crus en absence de ferments lactiques (jusqu'à 7 log₁₀ dénombré à 192h). La croissance d*'E. coli* a été inhibée en présence des ferments lactiques par comparaison aux fromages témoins. Cette inhibition est en grande partie liée à la production de l'acide lactique par le ferment Bionatif®, comme tout autre ferment acidifiant. Le ferment Bioprotecteur a provoqué une inhibition du nombre d*'E*. *coli* significativement plus élevée que le ferment Bionatif® après 20h (réduction de 2 log₁₀). Le nombre d*'E*. *coli* a diminué en dessous de la limite de dénombrement (<1 UFC/mL) après 192 h et est resté en dessous de ce niveau pendant toute la durée d'affinage des fromages. Le ferment Bioprotecteur peut donc contribuer à la sécurité alimentaire et à prolonger la durée de conservation de ce type de produits.

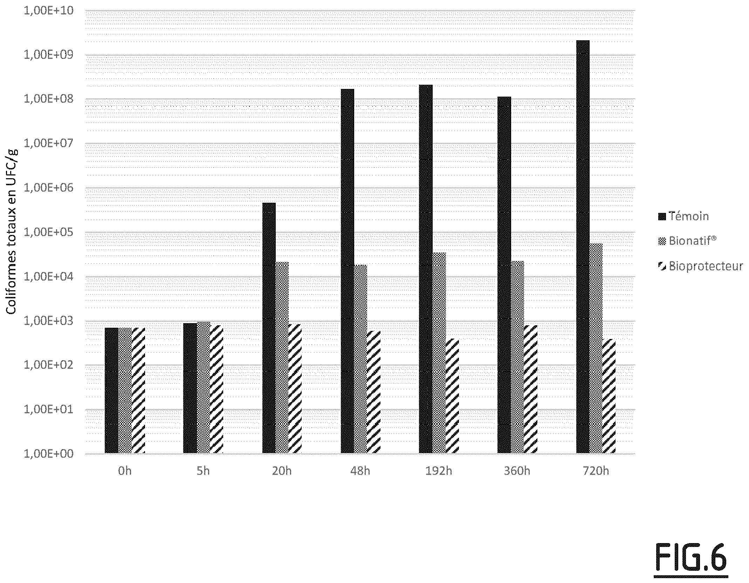

La **Figure 6** illustre le dénombrement des coliformes (UFC/g) sans et avec la présence d'un ferment issu du procédé Bionatif® ou un ferment Bioprotecteur ajouté lors de la fabrication d'un fromage pâte molle lactique.

Le lait cru utilisé contenait 710 UFC/mL de coliformes avant ensemencement des levains. Les *coliformes* se développent très bien dans les fromages aux laits crus en absence de ferments lactiques (jusqu'à 9 log₁₀ dénombré à 720h). La croissance des coliformes a été inhibée en présence des ferments lactiques par comparaison aux fromages témoins. Le ferment Bioprotecteur a provoqué une inhibition des coliformes significativement plus élevée que le ferment Bionatif® après 20h (réduction de 2 log₁₀). Cette réduction est maintenue pendant toute la durée d'incubation des fromages.

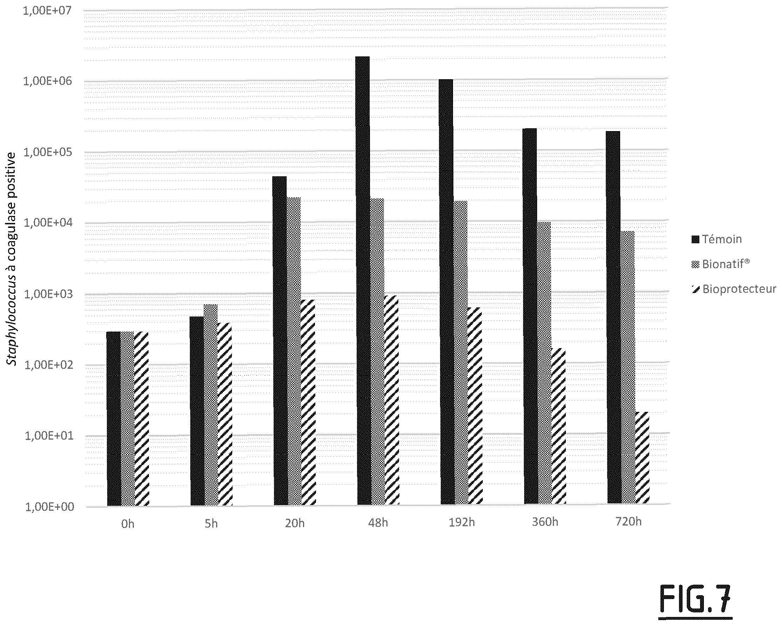

La **Figure 7** illustre le dénombrement des *S. aureus* (UFC/g) sans et avec la présence d'un ferment issu du procédé Bionatif® ou un ferment Bioprotecteur ajouté lors de la fabrication d'un fromage pâte molle lactique. La croissance des *Staphylococcus à coagulase positive* a été inhibée en présence des ferments lactiques par comparaison aux fromages témoins. Le ferment Bioprotecteur a provoqué une inhibition des *Staphylococcus* significativement plus élevée que le ferment Bionatif® après 20h (réduction de 2 log₁₀). Cette réduction est maintenue pendant toute la durée d'incubation des fromages (réduction de 3 log₁₀ après 720h).

## Revendications

1. Levain bioprotecteur comprenant plus de 99,00% de bactéries de l'espèce *Lactococcus lactis* en abondance relative, et obtenu par une méthode de préparation comprenant une étape de fermentation spontanée de lait natif de mammifère à une température comprise entre 20°C et 30°C, de préférence à 25°C, jusqu'à un pH d'arrêt de la fermentation compris entre 4,8 et 5,8, de préférence de 5,0.

2. Levain bioprotecteur selon la revendication 1, ledit levain ayant une activité bioprotectrice à l'encontre de la flore pathogène, en particulier à l'encontre de *Listeria spp., Salmonella spp.* et *Streptococcus aureus,* et de la flore d'altération, en particulier à l'encontre des coliformes, des laits natifs de mammifère.

3. Levain bioprotecteur selon la revendication 1 ou 2, comprenant entre 10⁸ ufc de bactéries /ml de levain et 10⁹ ufc de bactéries /ml de levain.

4. Levain bioprotecteur selon l'une quelconque des revendications 1 à 3, dans lequel la méthode de préparation inclut après l'étape de fermentation, une étape de report en congélation dudit levain et/ou une étape de concentration par repiquages répétés successivement au maximum 3 fois.

5. Levain bioprotecteur selon l'une quelconque des revendications 1 à 4, comprenant :
(i) 99,24% de bactéries de l'espèce *Lactococcus lactis* en abondance relative, 0,69% de bactéries du genre *Enterococcus* en abondance relative et 0,06% de bactéries du genre *Hafnia* en abondance relative,
(ii) 100,00% de bactéries de l'espèce *Lactococcus lactis* en abondance relative,
(iii) 99,90% de bactéries de l'espèce *Lactococcus lactis* en abondance relative et 0,10% de bactéries du genre *Macrococcus* en abondance relative, ou
(iv) Un mélange de (i) et (ii), de (i) et (iii), de (ii) et (iii), ou de (i), (ii) et (iii).

6. Méthode de préparation d'un levain à partir de lait natif de mammifère, ladite méthode comprenant :
a) une étape de fermentation spontanée de lait natif de mammifère, à une température comprise entre 20°C et 30°C, de préférence à 25°C, jusqu'à un pH d'arrêt de la fermentation à 6,
b) une étape d'ajout du levain bioprotecteur selon l'une des revendications 1 à 5 dans le lait fermenté obtenu à la fin de l'étape a), et
c) une étape de fermentation spontanée du lait fermenté obtenu à la fin de l'étape b), à une température comprise entre 20°C et 30°C, de préférence à 25°C, jusqu'à un pH d'arrêt de la fermentation compris entre 4,8 et 5,8, de préférence de 5,0.

7. Méthode de réduction ou d'élimination de la flore pathogène et de la flore d'altération d'un lait cru de mammifère, ladite méthode comprenant le mélange dudit lait cru avec le levain bioprotecteur selon l'une quelconque des revendications 1 à 5.

8. Utilisation du levain bioprotecteur selon l'une quelconque des revendications 1 à 5 dans la fabrication d'un fromage, d'un beurre, d'un lait fermenté ou d'une crème fermentée.

9. Méthode de préparation d'une morge comprenant le mélange de flores d'affinage avec le levain bioprotecteur selon l'une quelconque des revendications 1 à 5.

10. Levain bioprotecteur selon l'une quelconque des revendications 1 à 5 pour son utilisation comme probiotique.
